# EUROPEAN PATENT APPLICATION

(11) **EP 0 953 562 A1**
(43) Date of publication of application: **03.11.1999**
(21) Application number: 99107948.4
(22) Date of filing: 22.04.1999
(51) Int. Cl.: C07C 69/738, A61K 7/46

(54) **Beta, gamma-unsaturated delta-keto esters**

(30) Priority: 28.04.1998 EP 98107663
(71) Applicant: Givaudan Roure (International) S.A., 1214 Vernier-Genève (CH)
(72) Inventor: Anderson, Denise, 8032 Zürich (CH); Frater, Georg, 8400 Winterthur (CH)
(74) Representative: Buntz, Gerhard

(57) **Abstract**

The beta,gamma-unsaturated delta-keto esters of formula I are useful as precursors for the delivery of organoleptic compounds, especially for flavours, fragrances and masking agents, and/or of antimicrobial compounds.

## Description

The present invention relates to beta,gamma-unsaturated delta-keto esters and the use of beta, gamma-unsaturated delta keto esters as precursors for (a) organoleptic compounds, especially for flavours, fragrances, and/or (b) masking agents, and/or (c) antimicrobial compounds.

A principal strategy currently employed in imparting odours to consumer products is the admixing of a fragrance, flavour, masking agent or antimicrobial compound directly into the product to be treated. There are, however, several drawbacks to this strategy. The fragrance material can, for example, be too volatile and/or too soluble, resulting in fragrance loss during manufacturing, storage, and use. Many fragrance materials are also unstable over time. This again results in loss during storage.

In many consumer products it is desirable for the fragrance to be released slowly over time. Microencapsulation and inclusion complexes, especially with cyclodextrins, have been used to help decrease volatility, improve stability and provide slow-release properties. However, these methods are for a number of reasons often not successful. In addition, cyclodextrins can be too expensive.

Fragrance precursors for scenting fabrics being used in a washing cycle in the presence of a lipase-containing detergents are described in WO 95/04809. The fragrance precursors contained in the detergent and/or in the softener are cleaved by the lipase and a single odoriferous compound, either an odoriferous alcohol or aldehyde or ketone, is yielded. Thereby a prolonged scenting effect on the fabric is obtained.

An object of the present invention is to provide new precursors for organoleptic and/or antimicrobial compounds with different activities, especially as mentioned at the beginning.

A further object of the invention is to provide such new precursors which are stable under usual transport and storage conditions. A still further object of the present invention is to provide precursors supplying different active compounds simultaneously or successively.

It has now been found that beta, gamma-unsaturated delta-keto esters of the hereafter described formula I fulfil at least one of the afore mentioned tasks and, hence, are useful as precursors for the afore mentioned purposes: 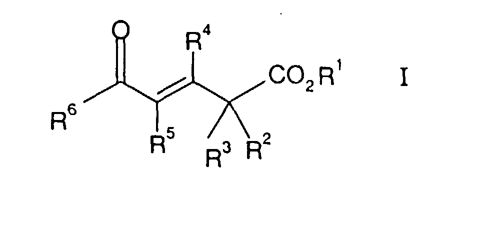 wherein
R¹ is a radical of either an alcohol R¹OH, or a radical of an alcohol R¹OH which radical further contains at least one remaining part of formula I;
R² to R⁶ are independently H or substituted or unsubstituted, branched or unbranched alkyl-, alkenyl-, akinyl-, cycloalkyl-, cycloalkenyl- or aromatic radicals, preferably with 1 to 10 C atoms, or
   R² or R³ is CO₂R¹;
and/or at least one of the pairs R²+R³, R³+R⁴, R³+R⁵, R⁴+R⁵, R⁴+R⁶ forms a saturated or unsaturated or aromatic ring with 3 to 7 C atoms, preferably with 5 to 6 C atoms, whereby this/these ring(s) can be further substituted by one or more alkyl- and/or alkenyl radicals and/or by one or more -CO₂R⁷ groups wherein R⁷ is a radical of an alcohol R⁷OH, whereby R⁷ is preferably R¹.

The alcohol R¹OH is preferably an organoleptic one.

The compounds of formula I are not limited to any particular stereoisomers, all possible stereoisomers (E/Z isomers, enantiomers, diastereomers) and all mixtures are thus included within the scope of the invention.

The compounds of formula I with a radical R¹ of an organoleptic alcohol R¹OH are new.

Of specific interest as precursors according to the present invention are compounds of the following types of formula I:
(3-oxo-2-pentyl-cyclopent-1-enyl)-acetic acid esters of R¹ (Ia),
2-methyl-4-oxo-3-pentyl-cyclopent-2-enecarboxylic acid esters of R¹ (Ib),
2-methyl-4-oxo-6-propyl-cyclohex-2-enecarboxylic acid esters of R¹ (Ic),
2-ethyl-5-oxo-pent-3-enoic acid esters of R¹ (Id),
5-oxo-5-(2,6,6-trimethyl-cyclohex-1-enyl)-pent-3-enoic acid esters of R¹(Ie),
(2-but-2-enoyl-3,3-dimethyl-cyclohex-1-enyl)-acetic acid esters of R¹ (If),
5,5-dimethyl-3-pent-4-enoyl-cyclohex-2-enecarboxylic acid esters of R¹ (Ig),
7-methyl-3-(2-oxo-ethylidene)-oct-6-enoic acid esters of R¹ (Ih),
5-methyl-2-(1-methyl-3-oxo-propenyl)-hex-4-enoic acid esters of R¹ (Ii),
1,1,2,3,3-pentamethyl-7-oxo-2,3,4,5,6,7-hexahydro-1H-indene-4-carboxylic acid esters of R¹(Ij),
(2-hydroxy-4-methyl-3-oxo-cyclopent-1-enyl)-acetic acid esters of R¹ (Ik),
6-isopropenyl-3-methyl-4-oxo-cyclohex-2-enecarboxylic acid esters of R¹ (Il),
3-isopropenyl-4a,5-dimethyl-7-oxo-1,2,3,4,4a,5,6,7-octahydro-naphthalene-1-carboxylic acid esters of R¹ (Im), and
3-hexyl-4-oxo-cyclopent-2-enecarboxylic acid esters of R¹ (In).

The following compounds are preferred precursors:
(3-oxo-2-pentyl-cyclopent-1-enyl)-acetic acid methyl ester,
(3-oxo-2-pentyl-cyclopent-1-enyl)-acetic acid 3,7-dimethyl-oct-6-enyl ester,
(3-oxo-2-pentyl-cyclopent-1-enyl)-acetic acid phenethyl ester,
(3-oxo-2-pentyl-cyclopent-1-enyl)-acetic acid 3,7-dinlethyl-octa-2,6-dienyl ester,
2-methyl-4-oxo-6-propyl-cyclohex-2-enecarboxylic acid ethyl ester,
2-methyl-4-oxo-6-propyl-cyclohex-2-enecarboxylic acid dec-9-enyl ester,
2-methyl-4-oxo-3-pentyl-cyclopent-2-ene carboxylic acid phenethyl ester,
2-(3-oxo-2-pentyl-cyclopent-1-enyl)-malonic acid didec-9-enyl ester,
2-(4-oxo-3-pentyl-cyclopent-2-enyl)-malonic acid bis-(3,7-dimethyl-oct-6-enyl) ester,
2-methyl-4-oxo-6-propyl-cyclohex-2-enecarboxylic acid 3,7-dimethyl-octa-2,6-dienyl ester, and
2-ethyl-5-oxo-pent-3-enoic acid ethyl ester.

It is herewith emphasized that preferably also compounds with at least one further CO₂R¹- group are used according to the invention. Examples are the known compound Io mentioned hereafter, which according to the invention acts as a precursor which after decomposition yields the well known fragrance dihydrojasmone, or the following compounds Ip to Ir:
2-(3-oxo-2-pentyl-cyclopent-1-enyl)-malonic acid esters of R¹ (Io),
5-(2-methoxycarbonylmethyl-6,6-dimethyl-cyclohex-1-enyl)-5-oxo-3-enoic acid methyl ester and other esters of R¹ (Ip),
2-(2-hydroxy-4-methyl-3-oxo-cyclopent-1-enyl)-malonic acid esters of R¹ (Iq),
2-(3-methyl-but-2-enyl)-3-(2-oxo-ethylidene)-pentanedioic acid diesters of R¹ (Ir).

Of course, there are still further preferred compounds of formula I which fulfil advantageously at least one of the afore mentioned objects of the invention, so e.g. compounds where the alcohol is strictly odourless, e.g. as derived from the preferred compound Is:

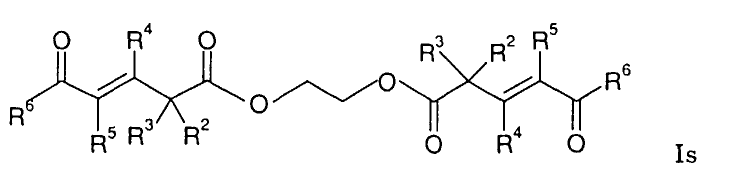

The compounds of formula I are mostly or nearly odourless at room temperature, atmospheric conditions and about 20 to 100% relative humidity. However, under activating conditions, they are cleaved and one or more active compounds, either ketones and/or aldehydes and/or alkohols, with organoleptic and/or antimicrobial properties are generated.

The activating conditions which lead to cleavage of the precursors and thereby to the liberating of the desired active compound(s) may comprise the presence of skin bacteria, especially axilla bacteria, or of an enzyme such as protease or lipase, or elevated temperature or acidic or alkaline pH-values or a combination of at least two of these activating conditions.

A compound of formula I, upon cleavage, provides at least one organoleptic ketone or aldehyde and optionally one or more alcohols having organoleptic and/or antimicrobial activity and therefore permits the development of useful consumer products with enhanced organoleptic and/or antimicrobial properties. The organoleptic ketones, aldehydes and alcohols obtained are useful as fragrances, flavours, masking agents and/or antimicrobial agents. Therefore, the invention relates to the use of all compounds of formula I as precursor's for organoleptic compounds, e.g. flavours, fragrances, masking agents, and/or as precursor's for antimicrobial agents.

The beta,gamma-unsaturated delta-keto esters of formula I can act as fragrance precursors in personal care products, in laundry products, cleaning compositions, pet care products and environment scents such as air fresheners. They can also act as precursors for odour masking agents in the same products as the fragrance precursors. They also can act as precursors for antimicrobial agents for these and further products. Further, they can act as flavour precursors in food, feed, tobacco and beverage products. The fragrance precursors and the precursors for odour masking agents as well as the flavour precursors of the invention may be used individually in an amount effective to enhance or to mask the characteristic odour or flavour of a material. More commonly, however, the compounds are mixed with other fragrance or flavour components in an amount sufficient to provide the desired odour or flavour characteristics. Any person skilled in the art will have knowledge how to make best use of the precursors of the invention.

Due, to the in situ generation of the active compounds the desired effect is prolonged and the substantivity on different substrates is enhanced. If two or more active compounds are provided, they can be generated, depending on the precursor and/or the, activating conditions, simultaneously or successively. Further, the precursors of the invention provide slow release of the active compounds.

Compounds of formula I may generate the following organoleptic ketones: 1-(2-cyclohexen)-2,4,4-trimethyl-but-2-enone*, carvone**,2-hexyl-cyclopent-2-en-1-one**, 2-pentyl-cyclopent-2-en-1-one, 3-methyl-2-pentyl-cyclopent-2-en-1-one**,2-hexylidenecyclopentanone*, 3,5-diethyl-5,6-dimethyl-2-cyclohexenone*, 4,4A,5,6,7,8-hexahydro-6-isopropenyl-4,4A-dimethyl-2(3H)-napthalenone**, 3-methyl-6-propylidenecyclohexanone*, 4-(1-methylethyl)-cyclohex-2-en-1-one, (E)-oct-3-en-2-one, 1-(2,3,4,7,8,8A-hexahydro-3,6,8,8-tetramethyl-1H-3A,7-methanoazulen-5-yl)-ethanone*, 2-hydroxy-3,5-dimethyl-cyclopent-2-ene-1-one*, 1-(3,3-dimethyl-1-cyclohexan-1-yl)ethanone*, 1-(2,4,6-trimethylcyclohex-3-en-1-yl)-but-1-en-3-one, acetylisolongifolene, 2-(3-methylbut-2-en-1-yl)-3-methyl-cyclopent-2-en-1-one, 2,6,6-trimethyl-1,3-cyclohexadienyl-1-carbaldehyde**, 3-methyl-5-(2,2,3-trimethylcyclopent-3-ene-1-yl)pent-3-ene-2-one*, 5-butylidene-2,2,4-trimethylcyclopentanone, 1,2,3,5,6,7-hexahydro-1,1,2,3,3-pentamethyl-4H-inden-4-one**, 3-methyl-5-propyl-cyclohex-2-en-1-one**, 4,4A,5,6,7,8-hexahydro-6-isopropyl-2(3H)-naphthalenone, 3,5,5-trimethyl-cyclohex-2-en-1,4-dione*, (E)-5-methyl-2-hepten-4-one, acetyl-diisoamylene**, dec-3-en-2-one, 2-ethyl-3,6,6-trinlethylcyclohex-2-enyl-but-2-en-1-one, 1(5,5-dimethyl-1(6)-cyclohexen-1-yl)-4-penten-1-one**, 1-(2,6,6-trimethyl-1-cyclohexen-1-yl)-but-2-ene-1-one**, 1-(2,6,6-trimethyl-2-cyclohexen-1-yl)-but-2-ene-1-one**, 1-(2,6,6,trimethyl-3-cyclohexen-1-yl)-but-2-ene-1-one**, 2,4,4,5,5-pentamethyl-1-cyclopentene-1-yl-ethanone*, whereby * indicates the preferred ketones and ** indicate the more preferred ketones.

Compounds of formula I may also generate the following organoleptic aldehydes:
2-methyl-4-(2,6,6-trimethyl-2-cyclohexen-1-yl)but-2-enalhex-2-enal*, 2-nonenal*, 2-tridecenal*, 3,7-dimethyl-oct-2,6-dien-1-al*, nonadienal*, 2,4-dimethyl-2,6-heptadienal, trans-dec-2-en-1-al*, 2,4-diethyl-hep-2,6-dien-1-al*, dodec-2-en-1-al*, 3,7-dimethyl-oct-2,6-dien-1-al*, 2,4-diethyl-hepta-2,6-dienal, 3₁7-dimethyl-nona-2,6-dien-1-al*, 3-propyl-2-hepten-1-al, 1-carboxaldehyde-4-(prop-2-en-2-yl)-cyclohex-1-ene, whereby * indicates the preferred aldehydes.

Examples of organoleptic monoalcohols constituting the residue R¹ in the compounds of formula I and generated upon cleavage are:
amyl alcohol, hexyl alcohol*, 2-hexyl alcohol*, heptyl alcohol*, octyl alcohol*, nonyl alcohol*, decyl alcohol*, undecyl alcohol*, lauryl alcohol*, myristic alcohol, 3-methyl-but-2-en-1-ol*, 3-methyl-1-pentanol, cis-3-hexenol*, cis-4-hexenol*, 3,5,5-trimethyl hexanol, 3,4,5,6,6-pentamethylheptan-2-ol*, citronellol*, geraniol*, oct-1-en-3-ol, 2,5,7-trimethyl octan-3-ol, 2-cis-3,7-dimethyl-2,6-octadien-1-ol, 6-ethyl-3-methyl-5-octen-1-ol*, 3,7-dimethyl-oct-3,6-dienol*, 3,7-dimethyloctanol*, 7-methoxy-3,7-dimethyl-octan-2-ol*, cis-6-nonenol*, 5-ethyl-2-nonanol, 6,8-dimethyl-2-nonanol*, 2,2,8-trimethyl-7 (8)-nonene-3-ol, nona-2,6-dien-1-ol, 4-methyl-3-decen-5-ol*, dec-9-en-1-ol, benzylalcohol, 2-methyl undecanol, 10-undecen-1-ol, 1-phenyl ethanol*, 2-phenyl ethanol*, 2-methyl-3-phenyl-3-propenol, 2-phenyl propanel*, 3-phenyl propanol*, 4-phenyl-2-butanol, 2-methyl-5-phenyl pentanol*, 2-methyl-4-phenyl-pentanol*, 3-methyl-5-phenyl-pentanol*, 2-(2-methylphenyl)-ethanol*, 4-(1-methylethyl)benzene methanol, 4-(4-hydroxyphenyl)butan-2-one*, 2-phenoxy ethanol*, 4-( 1-methylethyl)-2-hydroxy-1-methyl benzene, 2-methoxy-4-methyl phenol, 4-methyl phenol, anisic alcohol*, p-tolyl alcohol*, cinnamic alcohol*, vanillin*, ethyl vanillin*, eugenol*, isoeugenol*, thymol, anethol*, decahydro 2-naplithalenol, borneol*, cedrenol*, farnesol*, fenchyl alcohol*, menthol*, 3,7,11-trimethyl-2,6,10-dodecatrien-1-ol, alpha ionol*, tetrahydro ionol*, 2-(1,1-dimethylethyl)cyclohexanol*, 3-(1,1-dimethylethyl)cyclohexanol*, 4-(1,1-dimethylethyl)cyclohexanol*, 4-isopropyl cyclohexanol, 6,6-dimethyl-bicyclo [3.3.1]hept-2-ene-2-ethanol, 6,6-dimethyl-bicyclo [3.1.1]hept-2-ene-methanol*, p-menth-8-en-3-ol*, 3,3,5-trimethyl cyclohexanol, 2,4,6-trimethyl-3-cyclohexenyl-methanol*, 4-(1-methylethyl)cyclohexyl)methanol*, 4-(1,1-dimethylethyl)cyclohexanol, 2-(1,1-dimethylethyl)-cyclohexanol, 2,2,6-trimethyl-alpha-propyl cyclohexane propanol*, 5-(2,2,3-trimethyl-3-cyclopentenyl)-3-methylpentan-2-ol*, 3-methyl-5-(2,2,3-trimethylcyclopentyl-3-enyl)pent-4-en-2-ol*, 2-ethyl-4(2,2,3-trimethylcyclopentyl-3-enyl)but-2-en-1-ol*, 4-(5,5,6-trimethylbicyclo[2.2.1] hept-2-yl)-cyclohexanol*, 2-(2-methylpropyl)-4-hydroxy-4-methyl-tetrahydropyran*, 2-cyclohexyl propanol*, 2-(1,1-dimethylethyl)-4-methyl cyclohexanol*, 1-(2-tert-butyl-cyclohexyloxy)-2-butanol*, 1-(4-isopropyl-cyclohexyl)-ethanol*, 2,6-dimethyl-oct-7-en-2-ol**, 2,6-dimethyl-heptan-2-ol**, 3,7-dimethyl-octa-1,6-dien-3-ol**, whereby * indicates the preferred alcohols and ** indicate the more preferred alcohols.

Further, examples of polyalcohols constituting the residue R¹ in the compounds of formula I are:
diols such as: diethylene glycol, propylene glycol, triethylene glycol;
triols such as: glycerol;
sugars such as: furanoside and pyranoside sugars such as glucose, fructose;
polymers such as: hydroxyethylcellulose, hydroxypropylcellulose.

Compounds of formula I upon cleavage may also generate antimicrobial compounds. Examples of these compounds are e.g. presented by J.J. Kabara, Cosmet. Sci. Technol. Ser. (16) 1997, p 181-208, especially in Table 8.6.

Of course, the afore mentioned ketones, aldehydes, alcohols and antimicrobial compounds can serve mutually as fragrances, flavours, masking agents and antimicrobial compounds, respectively. A person of skill in the art is well aware of these interrelationships and can make use thereof to solve a specific problem by using the precursors of the present invention.

It is a matter of course, that it is not possible to give a complete list of the organoleptic and/or antimicrobial ketones, aldehydes and alcohols and polymeric alcohols which are generated as a result of the desired cleavage of the beta, gamma-unsaturated delta-keto esters of formula I by skin bacteria, by enzymes, by elevated temperatures or by acidic and/or alkaline pH-values. The skilled person is, however, quite aware of those ketones, aldehydes and alcohols which provide the desired organoleptic properties, e.g. for being used as fragrance, flavour or for odour masking, and/or showing antimicrobial effects.

The compounds of formula I may preferably be used as sustained release odorants and flavours but also to mask or attenuate undesirable odours or to provide additional odours not initially present in consumer products, i.e. personal care products such as cosmetic products destined for application to human skin such as underarm deodorants or antiperspirants or other deodorants contacting the body, or in hand lotions, hair care products such as shampoos and conditioners, baby powders, baby lotions, ointments, foot products, facial cleansers, body wipes, facial makeup, colognes, after-shave lotions, shaving creams, etc. Additional applications include laundry detergents, fabric softener's, fabric softener sheets, (automatic) dishwasher detergents and all purpose cleaners. Further applications are air fresheners and odorants, odour masking agents and/or antimicrobial agents.

The compounds I are also useful in the flavouring or aromatizing of food, especially of cooked food. Addition of the beta, gamma-unsaturated delta-keto esters either singly or as a mixture to a cake batter, e.g. a microwave cake batter, serves to impart appropriate baking aromas to the cake as it is heated in the microwave as well as impart flavouring in the finished product. Compounds I are also useful in the flavouring and aromatizing of beverages, e.g. hot beverages such as teas and instant beverages prepared by adding hot water to a powder. Compounds I can also act as slow release agents in acidic or alkaline beverages. Further, these compounds are also useful for flavouring and aromatizing tobacco products, e.g. cigarettes.

The amount required to produce the desired, overall effect varies depending upon the particular compounds of formula I chosen, the product in which it will be used, and the particular effect desired.

For example, depending upon the selection and concentration of the compound chosen, when a compound of the formula I is added either singly or as a mixture, e.g. to a deodorant or laundry product composition at levels ranging from about 0.1 to about 10 % by weight, or most preferred about 0.25 to about 4 % by weight, an odorant, i.e. an odoriferous ketone, aldehyde or both with or without one or more alcohols in an 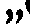 organoleptically effective amount" is released when the product is used. This newly formed odorant serves to enhance the odour of the product itself or of an fragrance present in the product.

Depending upon the selection and concentration, addition of the compounds I, either singly or as a mixture, to cigarette tobacco at levels ranging from about 5 ppm to about 50'000 ppm tends to enhance the smoking flavour and/or mask undesirable smoking odours. An important property of these compounds I is that the flavourant or odorant is covalently bound as a non-volatile compound and the flavourant or odorant is released only when the tobacco product is ignited and burns.

Addition of the compounds of formula I either separately or as a mixture at levels suitably ranging from about 5 ppm to about 50'000 ppm by weight onto the media enclosing the tobacco serves to incorporate the odorant/flavourant in the side-stream smoke of the tobacco. Air borne flavourants and/or odorants are thus introduced. This newly formed odorant or flavourant serves to enhance or mask the smoking odours depending upon selection and use levels of the compounds I.

As is evident from the above compilation of ketones, aldehydes and alcohols, a broad range of known odorants or flavours or mixtures can be generated from precursors of the invention. While manufacturing compositions the precursors of the invention may be used according to methods known to the perfumer, such as e.g. from W.A. Poucher, Perfumes, Cosmetics, Soaps, 2, 7th Edition, Chapman and Hall, London 1974.

The compounds of formula I can be prepared by using standard methods known to a person skilled in the art. A wide variety of methods for their preparation are known. Convenient methods are outlined in the Examples without limiting the invention thereto.

The present invention is further described by the following examples which are presented solely for the non-limiting purpose of further illustrating the invention.

### Example 1

### (3-Oxo-2-pentyl-cyclopent-1-enyl)-acetic acid methyl ester

A solution of 14 ml of 5.4 M sodium methoxide in 60 ml of methanol was heated to reflux, then 9.22 g of dimethyl malonate were dropped in. After that, a solution of 8.72 g of 3-methoxy-2-pentyl-2-cyclopenten-1-one (Katsube et al., Agr. Biol. Chem. (1969), 33 (7), 1078-86) in 20 ml of methanol was dropped in. After 31 hours of stirring under reflux, the reaction mixture was cooled down, acidified to pH 3 using conc. HCl and then refluxed for another 90 min. Then the methanol was evaporated, the residue was diluted with water, filtered, extracted with ether and washed with saturated sodium bicarbonate and water. The organic phase was dried, filtered and evaporated to dryness. The resulting yellow oil was purified by chromatography to yield 4.47 g of a yellow oil.
- NMR (CDCl₃) δ: 3.73 (s, 3H), 3.46 (s, 2H), 2.68-2.56 (m, 2H), 2.48-2.35(m, 2H), 2.24-2.11 (t, 2H), 1.50-1.14 (m, 6H), 0.94-0.80 (t, 3H).

### Example 2

### (3-Oxo-2-pentyl-cyclopent-1-enyl)acetic acid 3,7-dimethyl-oct-6-enyl ester

A mixture of 15.67 g (3-oxo-2-pentyl-cyclopent-1-enyl)-acetic acid methyl ester, 11.69 g citronellol and 0.41 g potassium tert. butoxide was stirred at 85°C and 170 Torr to distill off the methanol formed. After 5 hours the reaction mixture was cooled, diluted with ether and washed neutral with saturated sodium bicarbonate and water. The organic phase was dried, filtered and evaporated to dryness. The resulting yellow oil was purified by chromatography and Kugelrohr distillation to yield 12.66 g of a yellow oil.
- NMR (CDCl₃) δ: 5.08 (t, 1H), 4.17 (t, 2H), 3.43 (s, 2H), 2.68-2.53 (m, 2H), 2.48-2.33 (m, 2H), 2.19 (t, 2H), 2.09-1.88 (m, 2H), 1.69(s, 3H), 1.60 (s, 3H), 1.55-1.02 (m, 11H), 1.01-0.78 (m, 6H).

### Example 3

### (3-Oxo-2-pentyl-cyclopent-1-enyl)acetic acid phenethyl ester

According to the same procedure, (3-oxo-2-pentyl-cyclopent-1-enyl)acetic acid phenethyl ester was prepared from (3-oxo-2-pentyl-cyclopent-1-enyl)-acetic acid methyl ester, phenyl ethyl alcohol and potassium tert. butoxide.

### Example 4

### (3-Oxo-2-pentyl-cyclopent-1-enyl)acetic acid 3,7-dimethyl-octa-2,6-dienyl ester

According to the same procedure, (3-oxo-2-pentyl-cyclopent-1-enyl)acetic acid 3,7-dimethyl-octa-2,6-dienyl ester was prepared from (3-oxo-2-pentyl-cyclopent-1-enyl)-acetic acid methyl ester, geraniol and potassium tert. butoxide.

### Example 5

### 2-Methyl-4-oxo-6-propyl-cyclohex-2-enecarboxylic acid ethyl ester

In a 500 ml flask was placed 286.3 g (2.2 moles) of ethyl acetoacetate and 72.1 g (1 mole) of butyraldehyde The flask then was cooled in an ice-bath to 0°C and 1.6 g piperidine in 6 ml of ethanole was added with shaking. Then the flask was fitted with a bubbler, placed in a refrigerator and kept there for three days and a piperidine-ethanol mixture ( 1.6 g of piperidine, 6 ml of ethanol) was added each day (total 4.8 g of piperidine and 18 ml of ethanol). To ensure completeness of the reaction the mixture was allowed to stand for one day at room temperature. Then the mixture was refluxed at 110°C for six hours and then the piperidine, water and ethanol was distilled off. The residue was distilled (131°C, 0.16 mbar) to yield 181.5 g of a slightly yellow liquid.
- NMR (CDCl₄) δ: 5.95 (s, 1H); 4.1-4.3 (m, 2H); 3.1-3.25 (2xd, 1H); 2.55-2.7 (2xd, 1H); 2.0-2.54 (m, 2H); 1.95 (s, 3H); 1.12-1.5 (m, 7H); 0.8-1.0 (m, 3H)

### Example 6

### 2-Methyl-4-oxo-6-propyl-cyclohex-2-enecarboxylic acid dec-9-enyl ester

A mixture of 11.2 g 2-methyl-4-oxo-6-propyl-cyclohex-2-enecarboxylic acid ethyl ester, 15.7 g dec-9-ene-1-ol and 0.3 g potassium tert. butoxide was heated to reflux and the ethanol formed was distilled off. After 4 hours of stirring under reflux, the reaction mixture was cooled, washed with water, acidified using 2N HCl at 0°C and then extracted with ether. The organic phase was dried, filtered and evaporated to dryness. The resulting yellow oil was purified by chromatography to yield 13.4 g of a yellow oil.
- NMR (CDCl₃) δ: 6.03-5.94 (m, 1H), 5.94-5.70 (m, 1H), 5.08-4.88 (m, 2H), 4.24-4.03 (q, 2H), 3.30-3.05 (2d, 1H), 2.73-2.58 (2d, 1H), 2.58-2.15 (m, 2H), 2.15-2.01 (m, 2H), 1.98 (s, 3H), 1.78-1.54 (m, 2H), 1.53-1.15 (m, 14H), 1.05-0.78 (m, 3H).

### Example 7

### 2-Methyl-4-oxo-3-pentyl-cyclopent-2-ene carboxylic acid phenethyl ester

A mixture of 6.20 g of 2-methyl-4-oxo-3-pentyl-cyclopent-2-ene carboxylic acid ethyl ester (Demole E. et al., Helv. Chim. Ac., Vol. XLV, Fasciculus II (1962), No. 80-81, p. 685-692), 4.80 g phenethyl alcohol and 0.4 g tetraisopropyl-o-titanate was heated to 160°C and the ethanol formed was distilled off. After 2.5 hours, the reaction mixture was cooled, diluted with water, extracted with ether and washed with water. The organic phase was dried, filtered and evaporated to dryness. The resulting yellow oil was purified by chromatography to yield 4.24 g of a yellow oil.
- NMR (CDCl₃) δ: 7.40-7.12 (m, 5H), 4.43-4.29 (t, 2H), 3.63-3.47 (t, 1H), 3.03-2.89 (t, 2H), 2.56 (d, 2H), 2.25-2.07 (t, 2H), 1.91 (s, 3H), 1.55-1.12 (m, 6H), 0.97-0.78 (t, 3H).

### Example 8

### 2-(3-Oxo-2-pentyl-cyclonent-1-enyl)-malonic acid didec-9-enyl ester

In a first step malonic acid didec-9-enyl ester was prepared. To a mixture of 150 g dimethyl malonate and 464 g dec-9-en-1-ol, 30 g sodium methoxide (5.4 M in methanol) were dropped in. The mixture was heated to 120°C and the methanol formed was distilled off. After 6 hours, the mixture was cooled down, diluted with ether and washed with HCl 2N, saturated sodium bicarbonate and water. The organic phase was dried, filtered and evaporated to dryness. The resulting yellow oil was purified by distillation to yield 258 g of malonic acid didec-9-enyl ester, a yellow oil.

To a suspension of 2.25 g sodium hydride (60%) in 20 ml of THF, a solution of 21.31 g malonic acid didec-9-enyl ester in 20 ml of THF was dropped in at 0-5°C. After stirring for one hour, a solution of 11 g 3-chloro-2-pentyl-cyclopent-2-enone (Matschiner H. et al., Acta Chem. Scand. B 34 (1980) No, 2, p. 136) in 40 ml of THF was dropped in at room temperature. After stirring for 72 hours at room temperature, the reaction mixture was diluted with ether, washed with brine, HCl 2N and brine again. The organic phase was dried, filtered and evaporated to dryness. The resulting yellow oil was purified by chromatography to yield 7.3 g of a yellow oil.
- NMR (CDCl₃) δ: 5.93-5.68 (m, 2H), 5.08-4.87 (m, 4H), 4.75 (s, 1H), 4.29-4.03 (t, 4H), 2.85-2.68 (m, 2H), 2.48-2.38 (m, 2H), 2.30-2.11 (m, 2H), 2.10-1.93 (m, 4H), 1.80-1.52 (m, 4H), 1.51-1.13 (m, 26H), 0.98-0.78 (m, 3H).

### Example 9

### 2-(4-Oxo-3-pentyl-cyclopent-2-enyl)-malonic acid bis-(3,7-dimethyl-oct-6-enyl) ester

According to the same procedure as Example 8, 2-(4-oxo-3-pentyl-cyclopent-2-enyl)-malonic acid bis-(3,7-dimethyl-oct-6-enyl) ester was prepared from 3-chloro-2-pentyl-cyclopent-2-enone and malonic acid bis-(3,7-dimethyl-oct-6-enyl) ester.

### Example 10

### 2-Methyl-4-oxo-6-propyl-cyclohex-2-enecarboxylic acid 3,7-dimethyl-octa-2,6-dienyl ester

According to the same procedure as Example 6, 2-methyl-4-oxo-6-propyl-cyclohex-2-enecarboxylic acid 3,7-dimethyl-octa-2,6-dienyl ester was prepared from 2-methyl-4-oxo-6-propyl-cyclohex-2-enecarboxylic acid ethyl ester and citronellol.

### Example 11

### 2-Ethyl-5-oxo-pent-3-enoic acid ethyl ester

This compound was prepared according to the procedure of Khan and Peterson (*Tetrahedron Lett*. **1982**, 23, 2399.)

### Example 12

Test cloth was washed with a lipase-containing detergent to which one or more of the precursors of Examples 1-11 had been added. Headspace analysis of the wet and dry laundry indicated the presence of the fragrances. The fragrance level was higher than when the test cloth was washed with a lipase-containing detergent to which one or more fragrances were added.

### Example 13

Test cloth was washed with a lipase-containing detergent and then a fabric softener, containing one ore more of the precursors of Examples 1-11 was added to the rinse cycle. Headspace analysis of the wet and dry laundry indicated the presence of the fragrances. The fragrance level was higher than when the test cloth was washed with a lipase-containing detergent and then a fabric softener, containing one or more fragrances, was added to the rinse cycle.

### Example 14

Axilla bacteria cultures containing 0.1 % of one of each precursors of Examples 1-11 were incubated for 20 hours at 30°C. After filtration from the cells, the presence of the corresponding fragrance was in each case detected by headspace-GC techniques and/or the majority of an 18 member panel.

The same tests were carried out with inactivated cultures (85°C / 20 min). The odour of the corresponding fragrance could not be detected after incubation, excluding therefore a hydrolysis by the medium or the culture.

### Example 15

The following set forth examples for the use of the compounds of the present invention in various products. The methods of forming the following compositions are well known to those skilled in the art. All formulations may contain additional ingredients known to those skilled in the art, e.g. colorants, opacifiers, buffers, antioxidants, vitamins, emulsifiers, UV absorbers, silicones and the like. All products can also be buffered to the desired pH. All values are % w/w. Delayed Release Fragrances stands in the following for compounds of Examples 1-11.

### a) Deo-colognes

| | | | | |
|---|---|---|---|---|
| Delayed Release Fragrances | 0.5 | 1.5 | 2.5 | 6.0 |
| Fragrance | 0.5 | 1.5 | 2.5 | 6.0 |
| Triclosan (Ciby Geigy) | 1.0 | - | 0.75 | 1.0 |
| Alcohol to | 100 | 100 | 100 | 100 |

### b) Deo-Sticks

### Antiperspirant:

| | |
|---|---|
| Ethylene Glycol Monostearate | 7.0 |
| Shea butter | 3.0 |
| Neobee 1053 (PVO International) | 12.0 |
| Generol 122 (Henkel) | 5.0 |
| Kesscowax B (Akzo) | 17.0 |
| Dimethicone Dow Corning 345 | 35.0 |
| Aluminum Sesquichlorhydrate | 20.0 |
| Delayed Release Fragrances | 0.5 |
| Fragrance | 0.5 |

### Antiperspirant:

| | |
|---|---|
| Steary Alcohol | 17.0 |
| Castor Wax | 3.0 |
| Talc | 5.0 |
| Aluminum Zirconium Tetrachlorhydrate | 20.0 |
| Delayed Release Fragrances | 1.0 |
| Fragrance | 1.0 |
| Dimethicone Dow 245 | to 100.0 |

### Clear Deodorant Stick:

| | |
|---|---|
| Witconol APM | 43.0 |
| Propylene Glycol | 20.0 |
| Alcohol 39C | 20.0 |
| Demin Water | 7.0 |
| Monamid 150ADD | 5.0 |
| Millithix 925 | 2.0 |
| Ottasept Extra | 0.5 |
| Delayed Release Fragrances | 0.75 |
| Fragrance | 0.75 |

### Deodorant Stick:

| | |
|---|---|
| Propylene Glycol | 69.0 |
| Demin Water | 21.8 |
| Triclosan | 0.2 |
| Sodium Stearate | 8.0 |
| Delayed Release Fragrances | 0.5 |
| Fragrance | 0.5 |

### Alcohol free Deodorant Stick:

| | |
|---|---|
| PPC-3 Myristyl Ether (Witconol APM) | 36.0 |
| Propylene Glycol | 36.0 |
| Demin Water | 19.0 |
| Triclosan | 0.25 |
| Sodium Stearate | 7.75 |
| Delayed Release Fragrances | 0.5 |
| Fragrance | 0.5 |

### Antiperspirant Aerosol:

| | |
|---|---|
| Absolute Ethanol | 15.0 |
| Zirconium Aluminum Tetrachlorhydrate | 5.0 |
| Bentone 38 | 1.5 |
| Delayed Release Fragrances | 0.75 |
| Fragrance | 0.75 |
| S-31 Hydrocarbon propellant | to 100.0 |

### Antiperspirant Pump:

| | |
|---|---|
| Demin Water | 57.5 |
| Aluminum Sesquichlorhydrate | 20.0 |
| Triton X-102 (Union Carbide) | 2.0 |
| Dimethyl Isosorbide (ICI) | 20.0 |
| Delayed Release Fragrances | 0.25 |
| Fragrance | 0.25 |

### Roll-On:

| | |
|---|---|
| Dimethicone DC 354 (Dow Corning) | 69.0 |
| Bentone 38 | 10.0 |
| Rezal 36 GP (Reheis Chem. Co.) | 20.0 |
| Delayed Release Fragrances | 0.5 |
| Fragrance | 0.5 |

### Example 16

A 1% solution of one or more of the products of Examples 1-11 in ethanol was applied to cigarette papers to produce levels of 5-50'000 ppm of each flavourant. The paper was incorporated in cigarettes and, upon burning, released a fragrant odour.

In the above examples the following components were used:

| | |
|---|---|
| Triclosan | 5-chloro-2-(2,4-dichlorophenoxy)phenol |
| Neobee 1053 | glycerol tricaprate/caprylate |
| Generol 122 | soya sterol |
| Kesscowax B | cetyl alcohol and glycol polymer |
| Witconol APM | polypropylene glycol-3 myristyl ether |
| Monamid 150 ADD | cocoamide diethanolamine |
| Millithix 925 | dibenzylidene sorbitol |
| Ottasept Extra | quaternium 18 hectorite |
| Bentone 38 | quaternium 18 hectorite |
| Triton X-102 | octoxynol-13 |
| Dimethicone DC 354 | mixture of fully methylated linear siloxanepolymers end-blocked with trimethylsiloxy units |
| Rezal 36 GP | Aluminum zirconium tetrachlorohydrexglycine |

### Example 17

### a) Fabric softener of the ester quat type (4 x concentrate):

| ingredients | chemical name | % |
|---|---|---|
| | | |

| PHASE A | | |
|---|---|---|
| deionised water | | to 100.0 |
| MgCl₂ (saturated sol.) | Magnesium chloride | 1.0 |

| PHASE B | | |
|---|---|---|
| Rewoquat WE 18 | Di-(tallowcarboxyethyl)hydroxy ethyl methylammonium methosulfate | 15.0 |
| Genapol O 100 | Ethoxylated fatty alcohol C16-C18 10EO | 2.0 |
| antifoam DB 31 | | 0.5 |

| PHASE C | | |
|---|---|---|
| isopropyl alcohol | | 3.0 |
| preservative | | Qs |
| perfume | | Qs |

### preparation process:

While stirring and heating to 65° C part A was admixed, then part B which had been preheated to 65° C. After cooling to room temperature part C was added.

The pH value of the finished product was 2.60. It turned out that the recommended level of perfume is 1.0 %. Hence, delayed release fragrances of Examples 1-11 could be any part of this 1.0 %.

### b) Fabric softener of the ester quat type (1 x concentrate):

| ingredients | chemical name | % |
|---|---|---|
| | | |

| PHASE A | | |
|---|---|---|
| deionised water | | to 100.0 |

| PHASE B | | |
|---|---|---|
| Rewoquat WE 18 | Di-(tallowcarboxyethyl)hydroxy ethyl methylammoniummethosulfate | 6.0 |
| Dobanol 25-9 | Ethoxylated fatty alcohol C12-C15 9EO | 0.50 |
| antifoam DB 31 | | 0.10 |

| PHASE C | | |
|---|---|---|
| Myacide BT 30 | 2-bromo-2-nitropropane 1,3 diol | 0.03 |
| Proxel GXL | Benzisothiazolinone sodium salt | 0.02 |
| perfume | | Qs |

### preparation process:

While stirring and heating to 65° C, part A was admixed, then part B which had been preheated to 65° C. After cooling to room temperature part C was added.

The pH value of the finished product was 3.5. It turned out that the recommended level of perfume is 0.3 % and delayed release fragrances of Examples 1-11 could be any part of this 0.3 %.

## Claims

1. Use of compounds as precursors, yielding upon decomposition organoleptic compounds, masking agents and/or antimicrobial compounds, having the formula 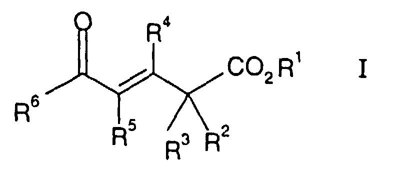 wherein
R¹ is a radical of either an alcohol R¹OH, or a radical of an alcohol R¹OH which radical further contains at least one remaining part of formula I;
R² to R⁶ are independently H or substituted or' unsubstituted, branched or unbranched alkyl-, alkenyl-, akinyl-, cycloalkyl-, cycloalkenyl- or aromatic radicals, preferably with 1 to 10 C atoms, or R² or R³ is CO₂R¹;
and/or at least one of the pairs R²+R³, R³+R⁴, R³+R⁵, R⁴+R⁵, R⁴+R⁶ forms a saturated or unsaturated or aromatic ring with 3 to 7 C atoms, preferably with 5 to 6 C atoms, whereby this/these ring(s) can be further substituted by one or more alkyl- and/or alkenyl radicals and/or by one or more -CO₂R⁷ groups wherein R⁷ is a radical of an alcohol R⁷OH.

2. Precursor of claim 1 wherein the radicals of R² to R⁶ have 1 to 10 C atoms.

3. Precursors of claim 1 or 2 wherein R⁷ is R¹.

4. Precursors of any one of the claims 1 to 3 wherein the alcohol R¹OH and/or R⁷OH is an organoleptic one.

5. Precursor of any of the claims 1 to 4 wherein R¹ and/or R⁷ is a radical derived from an alcohol R¹OH and/or R¹OH selected from the group consisting of amyl alcohol, hexyl alcohol*, 2-hexyl alcohol*, heptyl alcohol*, octyl alcohol*,nonyl alcohol*,decyl alcohol*,undecyl alcohol*, lauryl alcohol*, myristic alcohol, 3-methyl-but-2-en-1-ol*, 3-methyl-1-pentanol, cis-3-hexenol*, cis-4-hexenol*, 3,5,5-trimethyl hexanol, 3,4,5,6,6-pentamethylheptan-2-ol*, citronellol*, geraniol*, oct-1-en-3-ol, 2,5,7-trimethyl octan-3-ol, 2-cis-3,7-dimethyl-2,6-octadien-1-ol, 6-ethyl-3-methyl-5-octen-1-ol*, 3,7-dimethyl-oct-3,6-dienol*, 3,7-dimethyloctanol*, 7-methoxy-3,7-dimethyl-octan-2-ol*, cis-6-nonenol*, 5-ethyl-2-nonanol, 6,8-dimethyl-2-nonanol*, 2,2,8-trimethyl-7 (8)-nonene-3-ol, nona-2,6-dien-1-ol, 4-methyl-3-decen-5-ol*, dec-9-en-1-ol, benzylalcohol, 2-methyl undecanol, 10-undecen-1-ol, 1-phenyl ethanol*, 2-phenyl ethanol*, 2-methyl-3-phenyl-3-propenol, 2-phenyl propanol*, 3-phenyl propanol*, 4-phenyl-2-butanol, 2-methyl-5-phenyl pentanol*, 2-methyl-4-phenyl-pentanol*, 3-methyl-5-phenyl-pentanol*, 2-(2-methylphenyl)-ethanol*, 4-(1-methylethyl)benzene methanol, 4-(4-hydroxyphenyl)butan-2-one*, 2-phenoxy ethanol*, 4-(1-methylethyl)-2-hydroxy-1-methyl benzene, 2-methoxy-4-methyl phenol, 4-methyl phenol, anisic alcohol*, p-tolyl alcohol*, cinnamic alcohol*, vanillin*, ethyl vanillin*, eugenol*, isoeugenol*, thymol, anethol*, decahydro 2-naphthalenol, borneol*, cedrenol*, farnesol*, fenchyl alcohol*, menthol*, 3,7,11-trimethyl-2,6,10-dodecatrien-1-ol, alpha ionol*, tetrahydro ionol*, 2-(1,1-dimethylethyl)cyclohexanol)*, 3-(1,1-dimethylethyl)cyclohexanol*, 4-(1,1-dimethylethyl)cyclohexanol*, 4-isopropyl cyclohexanol, 6,6-dimethyl-bicyclo [3.3.1]hept-2-ene-2-ethanol, 6,6-dimethyl-bicyclo [3.1.1]hept-2-ene-methanol*, p-menth-8-en-3-ol*, 3,3,5-trimethyl cyclohexanol, 2,4,6-trimethyl-3-cyclohexenyl-methanol*, 4-(1-methylethyl)cyclohexyl-methanol*, 4-(1,1-dimethylethyl)cyclohexanol, 2-(1,1-dimethylethyl)-cyclohexanol, 2,2,6-trimethyl-alpha-prepyl cyclohexane propanol*, 5-(2,2,3-trimethyl-3-cyclopentenyl)-3-methylpentan-2-ol*, 3-methyl-5-(2,2,3-trimethylcyclopentyl-3-enyl)pent-4-en-2-ol*, 2-ethyl-4(2,2,3-trimethycyclopentyl-3-enyl)but-2-en-1-ol* 4-(5,5,6-trimethylbicyclo[2.2.1] hept-2-yl)-cyclohexanol*, 2-(2-methylpropyl)-4-hydroxy-4-methyl-tetrahydropyran*, 2-cyclohexyl propanol*, 2-(1,1-dimethylethyl)-4-methyl cyclohexanol*, 1-(2-tert-butyl-cyclohexyloxy)-2-butanol*, 1-(4-isopropyl-cyclohexyl)-ethanol*, 2,6-dimethyl-oct-7-en-2-ol**, 2,6-dimethyl-heptan-2-ol**, and 3,7-dimethyl-octa-1,6-dien-3-ol.**, whereby * indicates the preferred alcohols and ** indicate the more preferred alcohols.

6. Precursors of any one of the claims 1 to 5 yielding upon decomposition at least one organoleptic ketone selected from the group consisting of 1-(2-cyclohexen)-2,4,4-trimethyl-but-2-enone*, carvone**, 2-hexyl-cyclopent-2-en-1-one**, 2-pentyl-cyclopent-2-en-1-one, 3-methyl-2-pentyl-cyclopent-2-en-1-one**, 2-hexylidenecyclopentanone*, 3,5-diethyl-5,6-dimethyl-2-cyclohexenone*, 4,4A,5,6,7,8-hexahydro-6-isopropenyl-4,4A-dimethyl-2(3H)-napthalenone**, 3-methyl-6-propylidenecyclehexanone*, 4-(1-methylethyl)-cyclohex-2-en-1-one, (E)-oct-3-en-2-one, 1-(2,3,4,7,8,8A-hexahydro-3,6,8,8-tetramethyl-1H-3A,7-methanoazulen-5-yl)-ethanone*, 2-hydroxy-3,5-dimethylcyclopent-2-ene-1-one*, 1-(3,3-dimethyl-1-cyclohexen-1-yl)ethanone*, 1-(2,4,6-trimethylcyclohex-3-en-1-yl)-but-1-en-3-one, acetylisolongifolene, 2-(3-methylbut-2-en-1-yl)-3-methyl-cyclopent-2-en-1-one, 2,6,6-trimethyl-1,3-cyclohexadienyl-1-carbaldehyde**, 3-methyl-5-(2,2,3-trimethylcyclopent-3-ene-1-yl)pent-3-ene-2-one*, 5-butylidene-2,2,4-trimethylcyclopentanone, 1,2,3,5,6,7-hexahydro-1,1,2,3,3-pentamethyl-4H-inden-4-one**, 3-methyl-5-propyl-cyclohex-2-en-1-one**, 4,4A,5,6,7,8-hexahydro-6-isopropyl-2(3H)-naphthalenone, 3,5,5-trimethyl-cyclohex-2-en-1,4-dione*, (E)-5-methyl-2-hepten-4-one, acetyl-diisoamylene**, dec-3-en-2-one, 2-ethyl-3,6,6-trimethylcyclehex-2-enyl-but-2-en-1-one, 1-(5,5-dimethyl-1(6)-cyclohexen-1-yl)-4-penten-1-one**, 1-(2,6,6-trimethyl-1-cyclohexan-1-yl)-but-2-ene-1-one**, 1-(2,6,6-trimethyl-2-cyclehexen-1-yl)-but-2-ene-1-one**, 1-(2,6,6,trimethyl-3-cyclohexen-1-yl)-but-2-ene-1-one**, and 2,4,4,5,5-pentamethyl-1-cyclopentene-1-yl-ethanene*, whereby * indicates the preferred ketones and ** indicate the mere preferred ketones.

7. Precursors of any one of the claims 1 to 6 yielding upon decomposition at least one organoleptic aldehyde selected from the group consisting of hex-2-enal*, 2-nonenal*, 2-tridecenal*, 3,7-dimethyl-oct-2,6-dien-1-al*, nonadienal*, 2,4-dimethyl-2,6-heptadienal, trans-dec-2-en-1-al*, 2,4-diethyl-hep-2,6-dien-1-al*, dodec-2-en-1-al*, 3,7-dimethyl-oct-2,6-dien-1-al*, 2,4-diethyl-hepta-2,6-dienal, 3,7-dimethyl-nona-2,6-dien-1-al*, 3-propyl-2-hepten-1-al, and 1-carboxaldehyde-4-(prop-2-en-2-yl)-cyclohex-1-ene, whereby * indicates the preferred aldehydes.

8. Precursors of any one of the claims 5 to 7, whereby the alcohols, ketones and/or aldehydes serve as masking agents or antimicrobial compounds.

9. Precursors of any one of the claims 1 to 8 selected from the group consisting of (3-oxo-2-pentyl-cyclopent-1-enyl)-acetic acid esters of R¹ (Ia), 2-methyl-4-oxo-3-pentyl-cyclopent-2-enecarboxylic acid esters of R¹ (Ib), 2-methyl-4-oxo-6-propyl-cyclohex-2-enecarboxylic acid esters of R¹ (Ic), 2-ethyl-5-oxo-pent-3-enoic acid esters of R¹ (Id),
5-oxo-5-(2,6,6-trimethyl-cyclohex-1-enyl)-pent-3-enoic acid esters of R¹ (Ie), (2-but-2-enoyl-3,3-dimethyl-cyclohex-1-enyl)-acetic acid esters of R¹ (If, 5,5-dimethyl-3-pent-4-enoyl-cyclohex-2-enecarboxylic acid esters of R¹ (Ig), 7-methyl-3-(2-oxo-ethylidene)-oct-6-enoic acid esters of R¹(Ih),
5-methyl-2-(1-methyl-3-oxo-propenyl)-hex-4-enoic acid esters of R¹(Ii), 1,1,2,3,3-pentamethyl-7-oxo-2,3,4,5,6,7-hexahydro-1H-indene-4-carboxylic acid esters of R¹(Ij),
(2-hydroxy-4-methyl-3-oxo-cyclopent-1-enyl)-acetic acid esters of R¹ (Ik), 6-isopropenyl-3-methyl-4-oxo-cyclohex-2-enecarboxylic acid esters of R¹ (II) 3-isopropenyl-4a,5-dimethyl-7-oxo-1,2,3,4,4a,5,6,7-octahydro-naphthalene-1-carboxylic acid esters of R¹ (Im), and
3-hexyl-4-oxo-cyclopent-2-enecarboxylic acid esters of R¹ (In).

10. Precursor of any one of the claims 1 to 9 selected from the group consisting of (3-oxo-2-pentyl-cyclopent-1-enyl)-acetic acid methyl ester, (3-oxo-2-pentyl-cyclopent-1-enyl)-acetic acid 3,7-dimethyl-oct-6-enyl ester, (3-oxo-2-pentyl-cyclopent-1-enyl)-acetic acid phenethyl ester, (3-oxo-2-pentyl-cyclopent-1-enyl)-acetic acid 3,7-dimethyl-octa-2,6-dienyl ester, 2-methyl-4-oxo-6-propyl-cyclohex-2-enecarboxylic acid ethyl ester, 2-methyl-4-oxo-6-propyl-cyclohex-2-enecarboxylic acid dec-9-enyl ester, 2-methyl-4-oxo-3-pentyl-cyclopent-2-ene carboxylic acid phenethyl ester, 2-(3-oxo-2-pentyl-cyclopent-1-enyl)-malonic acid didec-9-enyl ester, 2-(4-oxo-3-pentyl-cyclopent-2-enyl)-malonic acid bis-(3,7-dimethyl-oct-6-enyl) ester, 2-methyl-4-oxo-6-propyl-cyclohex-2-enecarboxylic acid 3,7-dimethyl-octa-2,6-dienyl ester, and 2-ethyl-5-oxo-pent-3-enoic acid ethyl ester,

11. Precursor of claim 1 selected from group consisting of 2-(3-oxo-2-pentyl-cyclopent-1-enyl)-malonic acid esters of R¹, 5-(2-methoxycarbonylmethyl-6,6-dimethyl-cyclohex-1-enyl)-5-oxo-3-enoic acid methyl ester and other esters of R¹,
2-(2-hydroxy-4-methyl-3-oxo-cyclopent-1-enyl)-malonic acid esters of R¹, 2-(3-methyl-but-2-enyl)-3-(2-oxo-ethylidene)-pentanedioic acid diesters of R¹.

12. Precursors of claim 1 characterized by the formula

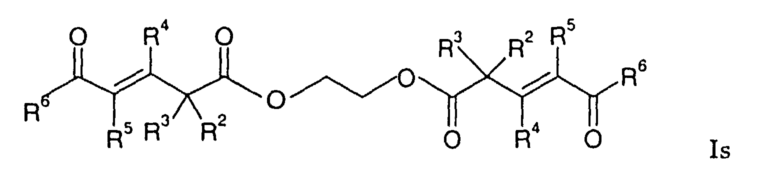

13. A composition for cosmetic application to the human skin, laundry products, detergents or fabric softeners and feed or feed or tobacco products containing at least one of the compounds according to any one of the claims 1 to 12.

14. A process for prolonging the effect of diffusion of the characteristic odour of an odoriferous compound on human skin or in laundry products, detergents or fabric softeners comprising applying at least one of the compounds of any one of the claims 1 to 12.

15. A method suppressing human body malodour by means of applying a composition of claim 13 to the human skin.

16. The use of at least one of the compounds of any one of the claims 1 to 12 as a fragrance precursor in a cosmetic composition or cosmetic product, laundry product, detergent or fabric softener or in a beverage, food, feed or tobacco product.

17. Beta,gamma-unsaturated delta keto esters having the formula 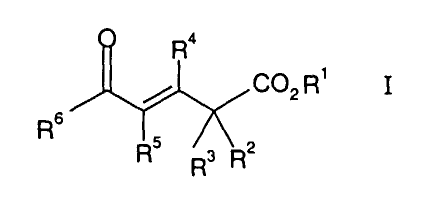 wherein
R¹ is a radical of an alcohol R¹OH;
R² to R⁶ are independently H or substituted or unsubstituted, branched or unbranched alkyl-, alkenyl-, akinyl-, cycloalkyl-, cycloalkenyl- or aromatic radicals, preferably with 1 to 10 C atoms, or
R² or R³ is CO₂R¹;
and/or at least one of the pairs R²+R³, R³+R⁴, R³+R⁵, R⁴+R⁵, R⁴+R⁶ forms a saturated or unsaturated or aromatic ring with 3 to 7 C atoms, preferably with 5 to 6 C atoms, whereby this/these ring(s) can be further substituted by one or more alkyl- and/or alkenyl radicals and/or by one or more -CO₂R⁷ groups wherein R⁷ is a radical of an alcohol R⁷OH and
wherein the alcohol(s) R¹OH and/or R⁷OH is/are organoleptic, or R¹ is a radical of an alcohol R¹OH which radical further contains at least one remaining part of formula I.

18. Beta,gamma-unsaturated delta-keto esters of claim 17 wherein R⁷ is R¹.

19. A beta,gamma-unsaturated delta-keto ester of claim 17 selected from the group consisting of
(3-oxo-2-pentyl-cyclopent-1-enyl)-acetic acid 3,7-dimethyl-oct-6-enyl ester,
(3-oxo-2-pentyl-cyclopent-1-enyl)-acetic acid 3,7-dimethyl-octa-2,6-dienyl ester,
(3-oxo-2-pentyl-cyclopent-1-enyl)-acetic acid phenethyl ester, 2-methyl-4-oxo-6-propyl-cyclohex-2-enecarboxylic acid dec-9-enyl ester, 2-methyl-4-oxo-3-pentyl-cyclopent-2-ene carboxylic acid phenethyl ester, 2-(3-oxo-2-pentyl-cyclopent-1-enyl)-malonic acid didec-9-enyl ester, 2-(4-oxo-3-pentyl-cyclopent-2-enyl)-malonic acid bis-(3,7-dimethyl-oct-6-enyl) ester, 2-methyl-4-oxo-6-propylcyclohex-2-enecarboxylic acid 3,7-dimethyl-octa-2,6-dienyl ester.
